(19) Europäisches Patentamt
European Patent Office
Office européen des brevets

(11) Publication number: **0 146 338**
**A2**

(12) # EUROPEAN PATENT APPLICATION

(21) Application number: **84308646.3**

(22) Date of filing: **12.12.84**

(51) Int. Cl.⁴: **A 61 K 31/12**, A 61 K 31/185, A 61 K 31/19

(30) Priority: **15.12.83 JP 237097/83**

(43) Date of publication of application: **26.06.85 Bulletin 85/26**

(84) Designated Contracting States: **AT BE CH DE FR GB IT LI LU NL SE**

(71) Applicant: **SHIRATORI PHARMACEUTICAL CO. LTD., 6-11-24 Tsudanuma, Narashino-shi Chiba-ken (JP)**

(72) Inventor: **Miyagaki, Michihiro, 23 Nakabiyomachi, Sakura-shi Chiba-ken (JP)**
Inventor: **Azuma, Hiroshi, 7-12-1-202, Negishidai, Asaka-shi Saitama-ken (JP)**
Inventor: **Katoh, Kunio, 112-3 Jonaicho, Sakura-shi Chiba-ken (JP)**

(74) Representative: **Evans, David Charles et al, F.J. CLEVELAND & COMPANY 40-43, Chancery Lane, London, WC2A 1JQ (GB)**

(54) **A blood accelerator 2, 3-diphosphoglyceric acid.**

(57) A blood 2,3-diphosphoglyceric acid accelerator which comprises as active ingredients thereof alpha-naphthoquinones or beta-naphthoquinones represented by the following general formula (I):

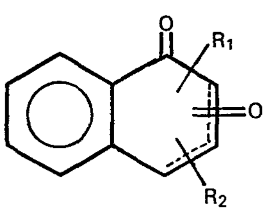

(I)

wherein $R_1$ represents a hydrogen atom, halogen atom, lower alkyl group or sulfon group, $R_2$ represents a hydrogen atom, halogen atom or hydroxycarbonylethylthio group, and the dotted line indicates a double bond at either position of 2 and 3 or 3 and 4.

The alpha- and beta-naphthoquinones elevate the concentration of flood 2,3-DPG which functions to increase the oxygen supply to the issue.

The accelerator agent according to the invention is especially usable for patients in a condition of decreasing 2,3-DPG, for example, after surgical operations or at the time of blood transfusion and for patients suffering from ischemis diseases.

EP 0 146 338 A2

ACTORUM AG

"A BLOOD 2,3-DIPHOSPHOGLYCERIC ACID ACCELERATOR"

DESCRIPTION

i) **Field of the Invention:**

This invention relates to a new blood 2,3-diphosphoglyceric acid accelerator, and more particularly, to a blood 2,3-diphosphoglyceric acid accelerator containing naphtoquinones as active ingredients.

ii) **Description of the Prior Art:**

A large amount of 2,3-diphosphoglyceric acid (2,3-DPG) is found in red blood cells of human being or certain mammals. 2,3-DPG is normally contained in a concentration of 4000 to 5000 n moles/ml RBC and is the majority of phosphate compounds in RBC. It is known that this 2,3-DPG is an intermediate metabolite of the glycolysis system, and functions to increase the oxygen supply to tissue by bonding itself with deoxyhemoglobin, changing the RBC pH value and decreasing remarkably the oxygen affinity of RBC [see Benesch, R et al: B.B.R.C. 26, 162 to 197 (1967), Chanutin, A et al: A.B.B.121 96 to 102 (1967)]. Therefore, if 2,3-DPG level in RBC is increased, even limited blood flow can supply the sufficient volume of oxygen to tissue.

The concentration of 2,3-DPG in RBC increases, owing to a conpensatory function, when the tissue lacks oxygen. In the ischemic diseases, however, it shows no increase but the decreasing trend even in the tissue of less oxygen. Such decrease of 2,3-DPG is also observed after a surgical operation or at a blood transfusion. In addition, 2,3-DPG in the preserved blood decreases as its preservation time progresses, and sometimes such blood becomes unusable due to remarkable decrease of the compound.

In such cases, if the oxygen supply can be enhanced by increasing 2,3-DPG in RBC, it would be clinically significant. However, administration of 2,3-DPG into tissue or blood induces no increase of 2,3-DPG in RBC, since 2,3-DPG does not permeate through the RBC membrane.

## SUMMARY OF THE INVENTION

The inventors carried out the study to enhance the concentration of 2,3-DPG in RBC, and consequently completed this invention by finding out the fact that alpha- and beta-naphtoquinones expressed by the following general formula (I) show the excellent activity of 2,3-DPG acceleration.

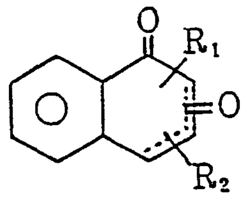

(I)

wherein $R_1$ represents a hydrogen atom, halogen atom, lower alkyl group or sulfon group, $R_2$ represents a hydrogen atom, halogen atom or hydroxycarbonylethylthio group, and the dotted line indicates a double bond at either position of 2 and 3 or 3 and 4.

Accordingly, an object of this invention is to provide a blood 2,3-diphosphoglyceric acid accelerator which comprises as active ingredients thereof alpha-naphthoquinones or beta-naphthoquinones represented by formula (I).

## DETAILED DESCRIPTION OF THE INVENTION AND PREFERRED EMBODIMENTS

Naphthoquinones used in this invention are, for example, 1,4-naphthoquinone, 2-methyl-1,4-naphthoqunone (vitamin $K_3$), 3-(1,4-dihydro-3-methyl-1,4-dioxo-2-naphthylthio)propionic acid (vitamin K-S (II)), 1,2-naphthoquinone-4-sulfonic acid, 2,3-dichloro-1,4-naphthoquinone, etc., and they are hypo-toxic substances as shown in Table 1.

Table 1

| Nephthoqunones | | Toxicities |
|---|---|---|
| 1,4-Naphthoquinone | $LD_{50}$ | 190 mg/kg rat, oral |
| Vitamin $K_3$ | $LD_{50}$ | 500 mg/kg, mouse, oral |
| 1,2-Naphthoquinone-4-sulfonic acid | $LD_{50}$ | 625 mg/kg, mouse intra-abdominal |
| 2,3-Dichloro-1,4-naphthoquinone | $LD_{50}$ | 1300 mg/kg, rat, oral |

The blood 2,3-DPG accelerator in this invention can be used singly or in combination with forming agents used generally for drug formulation, and made into powders, tablets, capsules, liquids or injection agents. The blood 2,3-DPG accelerator can stimulate the concentration of 2,3-DPG in RBC by direct addition in the preserved blood, oral administration or by injection.

The dose may be widely changeable depending on the symptom, but desirable doses are 10 $\mu$M to 10 mM for direct addition to blood and 20 $\mu$g to 10 mg/kg for the administration to human and animals.

The present invention will further be described by way of Examples.

**Example 1**

One hundred μM of each naphthoquinone (use 10 mM naphthoquinone dissolved in 50 % ethanol solution) was added to 1 ml of human ACD (acid chitorate dextrose) preserved blood (of which 2,3-DPG concentration had decreased to 1/20 of the normal level). After incubation for 60 min at 37°C, the reaction was stopped by addition of perchloric acid. According to Keitt's method (J. Lab. Clin. Med. 77, 470-475 (1971)), thereafter, 2,3-DPG was extracted and quantified. The results are shown in Table 2.

Table 2

| Naphthoquinones | Blood level of 2,3-DPG (n moles/ml RBC) |
|---|---|
| 1,4-naphthoquinone | 3554 |
| Vitamin $K_3$ | 3660 |
| Vitamin K-S (II) | 3700 |
| 1,2-naphthoquinone-4-sulfonic acid | 3923 |
| 2,3-dichloro-1,4-naphthoquinone | 3971 |
| Control (no addition of naphthoquinone) | 294 |

As stated in Table 2, the level of 2,3-DPG in RBC was remarkably enhanced by addition of naphthoquinones.

**Example 2**

The procedures in Example 1 was followed except addition of vitamin $K_3$ at various doses, and the relationship between the dose of vitamin $K_3$ and the concentration of 2,3-DPG was studied. The results are shown in Table 3.

Table 3

| Doses of Vitamin $K_3$ (μM) | Concentrations of 2,3-DPG (n moles/ml RBC) |
|---|---|
| 0 | 227 ± 15 (30) |
| 10 | 1124 ± 107 (30) |
| 100 | 3256 ± 244 (30) |
| 1000 | 5144 ± 253 (30) |
| 10000 | 5569 ± 311 (30) |

The figures in the parentheses indicate the numbers of test cases, and the results are described in the means ± standard errors.

Example 3

One hundred μM of vitamin $K_3$ was added in 1 ml of fresh blood taken from a patient with an ischemic disease, and the concentration of 2,3-DPG was determined in the same manner as in Example 1. The results are shown in Table 4.

Table 4

| Subjects | Concentrations of 2,3-DPG (n moles/ml RBC) | |
|---|---|---|
| | No addition of Vitamin $K_3$ | Addition of Vitamin $K_3$ |
| Buerger's Disease | 4253±287(12) | 5490±117(12) |
| Closed arteriosclerosis | 4796±365(15) | 5624±266(15) |
| Normal subjects | 4931± 53(75) | - |

(The figures in parentheses indicate the same numbers as mentioned in Table 3.)

As demonstrated in Table 4, the blood levels of 2,3-DPG in the patients with ischemic diseases were enhanced by the addition of vitamin $K_3$, and recovered to the normal level or higher levels.

CLAIMS

1. A blood 2,3-diphosphoglyceric acid accelerator which comprises as active ingredients thereof alpha-naphthoquinones or beta-naphthoquinones represented by the following general formula (I):

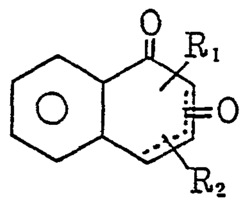

(I)

wherein $R_1$ represents a hydrogen atom, halogen atom, lower alkyl group or sulfon group, $R_2$ represents a hydrogen atom, halogen atom or hydroxycarbonylethylthio group, and the dotted line indicates a double bond at either position of 2 and 3 or 3 and 4.